(19) Europäisches Patentamt

European Patent Office

Office européen des brevets

(11) Veröffentlichungsnummer: **0 283 950**

**A2**

(12) # EUROPÄISCHE PATENTANMELDUNG

(21) Anmeldenummer: **88104337.6**

(22) Anmeldetag: **18.03.88**

(51) Int. Cl.⁴: **A23L 1/23 , A23L 1/235 , C12N 1/14**

---

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4019.

Patentansprüche für folgenden Vertragsstaat: ES.

(30) Priorität: **21.03.87 DE 3709342**

(43) Veröffentlichungstag der Anmeldung: **28.09.88 Patentblatt 88/39**

(84) Benannte Vertragsstaaten: **AT BE CH DE ES FR GB GR IT LI LU NL SE**

(71) Anmelder: **HOECHST AKTIENGESELLSCHAFT Postfach 80 03 20 D-6230 Frankfurt am Main 80(DE)**

(72) Erfinder: **Wink, Joachim, Dr. Bieberer Strasse 133 D-6050 Offenbach(DE)**
Erfinder: **Voelskow, Hartmut, Dr. Akazienstrase 22 D-6234 Hattersheim am Main(DE)**
Erfinder: **Grabley, Susanne, Dr. Hölderlinstrasse 7 D-6240 Königstein/Taunus(DE)**
Erfinder: **Deger, Hans-Matthias, Dr. Am Rheingauer Weg 8 D-6238 Hofheim am Taunus(DE)**

---

(54) **Pfirsich-Aroma, Verfahren zur Herstellung und seine Verwendung.**

(57) Mit Hilfe von Monilia fructicola DSM 4019 kann ein Pfirsicharoma hergestellt werden, das in wesentlichen aus γ-Octalacton, γ-Decalacton und Phenylethanol besteht. Bei Kultivierung des Pilzes in einem Nährmedium wird das Pfirsicharoma ausgeschieden.

EP 0 283 950 A2

Pfirsich-Aroma, Verfahren zur Herstellung und seine Verwendung

Fermentativ hergestellte Aromen aus Mikroorganismen gewinnen immer mehr an Bedeutung, da sie lebensmittelrechtlich als "natürliche Aromen" eingestuft werden. Dabei sind sowohl Reinsubstanzen als auch Gemische sehr gefragt. Bei dem natürlichen Aroma des Pfirsichs spielen $\gamma$-Lactone eine wesentliche Rolle. Schon seit Jahren bemüht man sich, diese Lactone mit Hilfe von Mikroorganismen zu gewinnen. Mit Hilfe einer Veilzahl von Hefestämmen, u.a. der Bäckerhefe Saccharomyces cerevisiae, ist es möglich, durch Biotransformation die Lactone aus den entsprechenden Säurevorstufen zu gewinnen (GB 916 822). Da man bei dieser Biotransformation jedoch von synthetischen Vorstufen ausgeht, können die entsprechenden Aromen bzw. Aromakomponenten nicht als "natürlich" bezeichnet werden.

Die mikrobielle Herstellung von $\gamma$-Decalacton wurde bereits mehrfach beschrieben, zum einen fermentativ durch Sporobolomyces odorus, hier jedoch mit sehr geringer Ausbeute, und zum anderen durch Fettspaltung und $\beta$-Oxidation mit Hilfe von Mikroorganismen wie Aspergillus oryzae, Candida rugosa und Candida lipolytica [US 4,560,656; S. Tahara et al., Agric. Biol. Chem. 36, 2585 (1972)].

Das Pfirsich-Aroma zeichnet sich jedoch durch eine Laktonmischung aus, wie sie, aber auch nur annähernd, mit Fusarium poae [J. Sarris, A. Latrasse, Agric. Biol. Chem 49 3227 (1985)] bzw. mit einigen Pityrosporum-Arten gewonnen werden kann (US 4,542,097). Ferner wurden die Bildung von Laktonen durch den Basidiomyceten Polyporus durus beschrieben [R. G. Berger et al., Naturforschung 41C, 963 (1986)]. Der große Nachteil bei diesem Verfahren ist jedoch die lange Fermentationszeit mit relativ niedrigen Ausbeuten.

Es wurde nun überraschend gefunden, daß der Pilz Monilia fructicola nach kurzer Fermentationszeit ein kräftig fruchtiges Pfirsicharoma produziert. Der Pilz wurde bei der Deutschen Sammlung von Mikroorganismen unter der Nummer DSM 4019 hinterlegt. Monilia fructicola DSM 4019 bildet sowohl das $\gamma$-Octalacton als auch das $\gamma$-Dekalacton, aber auch Phenylethanol sowie eine Anzahl weiterer $\gamma$-und $\delta$-Lactone, die zusammen ein abgerundetes Pfirsicharoma ergeben.

Die Erfindung betrifft somit:

1. Ein Pfirsich-Aroma erhältlich durch Fermentation von Monilia fructicola DSM 4019.

2. Ein Verfahren zur Herstellung eines Pfirsicharomas, das dadurch gekennzeichnet ist, daß Monilia fructicola DSM 4019 in einem Nährmedium kultiviert wird, bis das Pfirsicharoma in das Nährmedium ausgeschieden wird.

3. Die Verwendung des Pfirsicharomas zur Aromatisierung von Lebensmitteln und Kosmetikartikeln.

Im folgenden wird die Erfindung detailliert, insbesondere in ihren bevorzugten Ausführungsformen, beschrieben. Ferner wird die Erfindung in den Ansprüchen definiert.

Monilia fructicola DSM 4019 wurde aus einer Erdprobe aus dem Werk Hoechst AG, Frankfurt/Main isoliert. Die Erdprobe wurde in Wasser suspendiert und auf Agarplatten ausgestrichen. Einzelne Pilzkolonien wurden isoliert und weiter bearbeitet. Der Stamm Monilia fructicola DSM 4019 fiel dabei gegenüber den häufig vorkommenden Penicillium-und Aspergillusarten durch seine geringe Versporung auf.

Zur Durchführung des erfindungsgemäßen Verfahrens kann man nicht nur den Stamm DSM 4019 einsetzen, sondern auch dessen Mutanten und Varianten, soweit sie ein Pfirsicharoma produzieren. Solche Mutanten können in an sich bekannter Weise durch physikalische Mittel, beispielsweise Bestrahlung, wie mit Ultraviolett-oder Röntgenstrahlen, oder chemische Mutagene, wie beispielsweise Ethansulfonsäuremethylester (Ethylmethylsulfonat, EMS), 2-Hydroxy-4-methoxybenzophenon (MOB) oder N-Methyl-N'-nitro-N-nitrosoguanidin (MNNG), erzeugt werden.

Als bevorzugte Kohlenstoffquellen für die aerobe Fermentation eignen sich assimilierbare Kohlenhydrate und Zuckeralkohole, wie Glukose, Saccharose, Fructose, Sorbit und Mannit, sowie kohlenhydrathaltige Naturprodukte, wie Malzextrakt oder Kartoffelextrakt. Als stickstoffhaltige Nährstoffe kommen beispielsweise in Betracht: Aminosäuren, Peptide und Proteine sowie deren Abbauprodukte, wie Peptone oder Tryptone, ferner Fleischex rakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anderen anorganischen Salzen kann die Nährlösung beispielsweise Chloride, Carbonate, Sulfate oder Phosphate der Alkali-oder Erdalkalimetalle, Eisen, Zink und Mangan enthalten.

Die Bildung des Pfirsicharomas verläuft besonders gut in einer Nährlösung, die etwa 0,2 bis 5 %, bevorzugt 1 bis 4 %, Malzextrakt, 0.02 bis 0.5 %, bevorzugt 0,1 bis 0,4 % , Hefeextrakt, 0,1 bis 5 %, bevorzugt 0,5 bis 2 %, Glukose und 0,005 bis 0,2 %, bevorzugt 0,01 bis 0,1 %, Ammoniumsalz enthält, jeweils bezogen auf das Gewicht der gesamten Nährlösung. Insbesondere vorteilhaft ist die Zugabe von Triglyceriden, einzeln oder in Gemischen, wobei die Fettsäuren eine Kettenlänge bis zu 15 C-Atomen, bevorzugt 8 bis 10 C-Atome, besitzen und sich innerhalb der Moleküle durch ihre Kettenlänge unterscheiden können. Insbesondere bewährt hat sich die Zugabe von Triglyceriden des fraktionierten Kokosnußölfetts, deren Fettsäuren eine Kettenlänge von 8 und 10 C-Atomen haben. Die Kohlenstoffketten der Triglyceride dienen als direkte Vorstufe für die Lactone. Ihre Zusammensetzung beeinflußt so die quantitative Verteilung der einzelnen Lactone im Aromaspektrum.

Die Fermentation erfolgt aerob, in Oberflächenkultur oder submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 35°C, vorzugsweise bei etwa 25 bis 30°C, insbesondere bei 28 bis 30°C erfolgen. Der pH-Bereich sollte zwischen 6 und 8 liegen, vorteilhaft zwischen 6,5 und 7,5. Unter diesen Bedingungen läßt sich in der Kulturbrühe im allgemeinen nach 1 bis 4 Tagen ein nennenswerter Pfirsich-Geruch feststellen.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, überimpft werden.

Als Aroma kann der geklärte Fermentationsansatz direkt eingesetzt werden. Das Aroma kann aber auch nach an sich bekannten Methoden, beispielsweise mit Hilfe eines unpolaren Lösungsmittels bzw. Lösungsmittelgemisches , wie z.B. Pentan/Methylenchlorid, extrahiert werden oder an ein entsprechendes Trägermaterial adsorbiert, anschließend eluiert und destilliert werden.

Ein derart hergestelltes Pfirsicharoma setzt sich im wesentlichen aus γ-Octalacton, γ-Decalacton und Phenylethanol zusammen.

In dem folgenden Beispiel wird die Erfindung in ihren Einzelheiten erläutert. Prozentangaben beziehen sich auf das Gewicht, wenn nicht anders angegeben.

Beispiel

Vegetatives Mycel von Monilia fructicola DSM 4019 wird 2 bis 3 Tage bei 25°C in folgendem Medium inkubiert:

20 g/l Malzextrakt
2 g/l Hefeextrakt
10 g/l Glucose
0,5 g/l $(NH_4)_2HPO_4$

Diese so erhaltene Vorkultur dient nach Homogenisierung zum Animpfen der Hauptkultur, der ebenfalls das genannte Medium zugrunde liegt. Es wird mit 5 % Vorkultur, bezogen auf das Gewicht des Hauptkulturmediums, beimpft. Die Fermentation erfolgt bei 28°C unter kräftiger Belüftung (ca. 1 Fermentervolumen/min.) und Rühren. Nach 3 bis 5 Tagen erreicht der Stamm sein Maximum an Biomasse und Aromastoffen. Die Fermentation wird beendet und der gesamte Inhalt mit dem gleichen Volumen Pentan/Methylenchlorid (2:1 V/V) extrahiert. Nach Phasentrennung werden die Aromastoffe durch Destillation gewonnen. Die Ausbeute - schwankt zwischen 200 mg und 1 g/l.

Das Pfirsicharoma setzt sich im wesentlichen wie folgt zusammen:
50 % γ-Octalacton, 25 % γ-Decalacton und 25 % Phenylethanol, sowie Spuren weiterer γ-und δ-Lactone.

## Ansprüche

1. Pfirsicharoma erhältlich durch aerobe Kultivierung von Monilia fructicola DSM 4019, sowie dessen Varianten und Mutanten.

2. Pfirsicharoma nach Anspruch 1, im wesentlichen bestehend aus γ-Octalacton, γ-Decalacton und Phenylethanol.

3. Verfahren zur Herstellung eines Pfirsicharomas, dadurch gekennziechnet, daß Monilia fructicola DSM 4019 sowie dessen Varianten und Mutanten submers oder in Oberflächenkultur in einem Nährmedium kultiviert werden, bis das Pfirsicharoma in das Nährmedium ausgeschieden wird.

4. Verfahren nach Anspruch 3, dadurch gekennziechnet, daß das Nährmedium 0,2 bis 5 % Malzextrakt, 0,02 bis 0.5 % Hefeextrakt, 0,1 bis 5 % Glukose und 0,005 bis 0,2 % Ammoniumsalz, jeweils bezogen auf das Gewicht des Nährmediums, enthält.

5. Verfahren nach Anspruch 4, dadurch gekennzeichnet, daß das Nährmedium 1 bis 4 % Malzextrakt, 0,1 bis 0,4 % Hefeextrakt, 0,5 bis 2 % Glukose und 0,01 bis 0,1 % Ammoniumsalz, jeweils bezogen auf das Gewicht des Nährmediums, enthält.

6. Verfahren nach einem oder mehreren der Ansprüche 3 bis 5, dadurch gekennzeichnet, daß dem Nährmedium Triglyceride mit einer Fettsäure-Kettenlänge bis zu 15 C-Atomen zugesetzt werden.

7. Verfahren nach Anspruch 6, dadurch gekennzeichnet, daß die Kettenlänge der Fettsäure 8 bis 10 C-Atome beträgt.

8. Verwendung des Pfirsicharomas nach Anspruch 1 oder 2 zur Aromatisierung von Lebensmitteln und Kosmetikartikeln.

9. Monilia fructicola DSM 4019, dessen Varianten und Mutanten, sofern sie ein Pfirsicharoma produzieren.

Patentansprüche für den folgenden Vertragsstaat: ES

1. Verfahren zur Herstellung eines Pfirsicharomas, dadurch gekennzeichnet, daß Monilia fructicola DSM 4019 sowie dessen Varianten und Mutanten submers oder in Oberflächenkultur in einem Nährmedium kultiviert werden, bis das Pfirsicharoma in das Nährmedium ausgeschieden wird.

2. Verfahren nach Anspruch 1, dadurch gekannzeichnet, daß das Nährmedium 0,2 bis 5 % Malzextrakt, 0,02 bis 0.5 % Hefeextrakt, 0,1 bis 5 % Glukose und 0,005 bis 0,2 % Ammoniumsalz, jeweils bezogen auf das Gewicht des Nährmediums, enthält.

3. Verfahren nach Anspruch 2, dadurch gekannzeichnet, daß das Nährmedium 1 bis 4 % Malzextrakt, 0,1 bis 0,4 % Hefeextrakt, 0,5 bis 2 % Glukose und 0,01 bis 0,1 % Ammoniumsalz, jeweils bezogen auf das Gewicht des Nährmediums, enthält.

4. Verfahren nach einem oder mehreren der Ansprüche 1 bis 3, dadurch gekennzeichnet, daß dem Nährmedium Triglyceride mit einer Fettsäure-Kettenlänge bis zu 15 C-Atomen zugesetzt werden.

5. Verfahren nach Anspruch 4, dadurch gekennzeichnet, daß die Kettenlänge der Fettsäure 8 bis 10 C-Atome beträgt.

6. Verfahren nach einem oder mehreren der Ansprüche 1 bis 5, dadurch gekennzeichnet, daß das Pfirsicharoma im wesentlichen besteht aus $\gamma$-Octalacton, $\gamma$-Decalacton und Phenylethanol.

7. Verwendung des Pfirsicharomas, erhältlich nach einem der Ansprüche 1 bis 6 zur Aromatisierung von Lebensmitteln und Kosmetikartikeln.